# FASCICULE DE BREVET EUROPEEN

(11) **EP 2 983 797 B1**
(45) Date de publication et mention de la délivrance du brevet: **10.03.2021**
(21) Numéro de dépôt: 14720194.1
(22) Date de dépôt: 09.04.2014
(51) Int. Cl.: A61Q 19/08, A61K 8/99, A61K 8/31, A61Q 17/04, A61Q 19/00, A61P 17/18, A61P 39/06, A61P 43/00

(54) **COMPOSITION COSMETIQUE, PHARMACEUTIQUE OU ALIMENTAIRE COMPRENANT UN EXTRAIT RICHE EN CAROTENOIDES D'ARTHROBACTER AGILIS**
KOSMETISCHE, PHARMAZEUTISCHE ODER NAHRUNGSMITTELZUSAMMENSETZUNG MIT KAROTINOIDHALTIGEM EXTRAKT AUS ARTHROBACTER AGILIS
COSMETIC, PHARMACEUTICAL OR FOOD COMPOSITION COMPRISING CAROTENOID CONTAINING EXTRACT OF ARTHROBACTER AGILIS

(30) Priorité: 09.04.2013 FR 1353200
(43) Date de publication de la demande: 17.02.2016
(73) Titulaire: Thorel, Jean-Noël, 75014 Paris (FR)
(72) Inventeur: THOREL, Jean-Noël, F-75014 Paris (FR); PELLAY, François-Xavier, F-92360 Meudon La Foret (FR)
(74) Mandataire: Cabinet Laurent & Charras
(86) Numéro de dépôt international: PCT/FR2014/050852
(87) Numéro de publication internationale: WO 2014/167247

(56) Documents cités:
- WO-A1-00/24369
- JP-A- H07 132 096
- N. J. C. FONG ET AL: "Carotenoid accumulation in the psychrotrophic bacterium Arthrobacter agilis in response to thermal and salt stress", APPLIED MICROBIOLOGY AND BIOTECHNOLOGY, vol. 56, no. 5-6, 1 septembre 2001 (2001-09-01), pages 750-756, XP055092879, ISSN: 0175-7598, DOI: 10.1007/s002530100739
- DIESER M, GREENWOOD M, FOREMAN C M: "Carotenoid Pigmentation in Antarctic Heterotrophic Bacteria as a Strategy to Withstand Environmental Stresses", ARCTIC, ANTARCTIC AND ALPINE RESEARCH, vol. 42, no. 4, 1 mars 2010 (2010-03-01), - 31 décembre 2010 (2010-12-31), pages 396-405, XP009175105, Colorado, USA DOI: 10.1657/1938-4246-42.4.396
- DATABASE WPI Week 201249 Thomson Scientific, London, GB; AN 2012-H70470 XP002717875, & KR 2012 0068367 A (LG HOUSEHOLD&HEALTHCARE LTD) 27 juin 2012 (2012-06-27)
- KRINSKY ET AL: "Antioxidant functions of carotenoids", FREE RADICAL BIOLOGY AND MEDICINE, ELSEVIER SCIENCE, US, vol. 7, no. 6, 1 janvier 1989 (1989-01-01) , pages 617-635, XP025591958, ISSN: 0891-5849, DOI: 10.1016/0891-5849(89)90143-3 [extrait le 1989-01-01]
- MECKEL R A ET AL: "EXTRACTABILITY OF CAROTENOID PIGMENTS FROM NON-PHOTOSYNTHETIC BACTERIA WITH SOLVENTS AND DETERGENTS: IMPLICATIONS FOR THE LOCATION AND BINDING OF THE PIGMENTS", JOURNAL OF GENERAL MICROBIOLOGY, SOCIETY FOR MICROBIOLOGY, READING, GB, vol. 120, no. 1, 1 janvier 1980 (1980-01-01), pages 111-116, XP001147977, ISSN: 0022-1287
- SAITO T ET AL: "Hydroxyl radical scavenging ability of bacterioruberin", RADIATION PHYSICS AND CHEMISTRY, ELSEVIER SCIENCE PUBLISHERS BV, AMSTERDAM NL, vol. 50, no. 3, 1 septembre 1997 (1997-09-01), pages 267-269, XP004094273, ISSN: 0969-806X, DOI: 10.1016/S0969-806X(97)00036-4
- SAPERSTEIN S ET AL: "Association of carotenoid pigments with protein components in non-photosynthetic bacteria", BIOCHIMICA ET BIOPHYSICA ACTA, ELSEVIER, NL, vol. 16, 1 janvier 1955 (1955-01-01), pages 482-488, XP025667425, ISSN: 0006-3002, DOI: 10.1016/0006-3002(55)90267-5 [extrait le 1955-01-01]
- Cathrin Koch ET AL: "Reclassification of Micrococcus agilis (Ali-Cohen 1889) to the Genus Arthrobacter as Arthrobacter agilis comb. nov. and Emendation of the Genus Arthrobacter", INTERNATIONAL JOURNAL OF SYSTEMATIC BACTERIOLOGY Copyright, 1 octobre 1995 (1995-10-01), pages 837-839, XP055092874, Extrait de l'Internet: URL:http://ijs.sgmjournals.org/content/45/ 4/837.full.pdf [extrait le 2013-12-11]
- DATABASE GNPD [Online] MINTEL; 1 novembre 2004 (2004-11-01), Ecco Bella Botanicals: "M.D Formulated Age Antidote Day Cream", XP002732011, Database accession no. 10194702
- DATABASE GNPD [Online] MINTEL; 1 avril 2010 (2010-04-01), Bolaiya Cosmetics: "Shining Star Eye Cream", XP002732012, Database accession no. 1305663
- DATABASE GNPD [Online] MINTEL; 1 janvier 2010 (2010-01-01), Parafarm: "Dietary Supplement Planeta M Vikal'gin Laminariya Biologicheski Aktivnaya Dobavka", XP002732013, Database accession no. 1230777

## Description

### Domaine de l'invention

La présente demande décrit un extrait d'*Arthrobacter agilis* pour son utilisation notamment en cosmétique, et plus précisément un extrait riche en caroténoïdes.

Plus précisément, elle rapporte la mise en évidence de la protection des protéines contre les radicaux libres de l'oxygène (ROS) et les radiations de la lumière (UV et visible) d'un tel extrait, permettant de lutter contre l'altération des cellules, en particulier celle de la peau, provoquée par les agressions extérieures. Un tel extrait, en association avec la vitamine E ou un de ses dérivés utilisés comme agents antioxydants, permet donc de lutter contre le stress oxydant et notamment le vieillissement cellulaire.

### Etat de la technique

Chez les organismes aérobies et notamment chez l'homme, l'oxygène est nécessaire à la survie mais est également responsable de dommages oxydatifs liés à ses métabolites réactifs. Ainsi, le terme ROS (pour « reactive oxygen species ») ou ROM (pour « reactive oxygen metabolites ») est couramment utilisé pour désigner tous les radicaux libres et les espèces chimiques non radicalaires qui prennent part aux processus biologiques oxydatifs et dont l'excès est considéré comme la base d'un nombre croissant de procédés dégénératifs et de maladies. Plus précisément, le terme ROS inclut le radical anionique superoxyde O₂⁻·, le radical hydroxyle OH·, l'oxygène singulet ¹O₂ et le peroxyde d'hydrogène H₂O₂, ainsi que les radicaux alkoxys RO· et les radicaux peroxys ROO·, qui sont formés à partir des molécules organiques dans les processus oxydatifs. La production d'origine exogène de ces métabolites dépend essentiellement d'évènements physiques, tels que les irradiations aux ultraviolets (UV), ou des événements chimiques, tels que des agents xénobiotiques. La production endogène, quant à elle, est essentiellement liée à la fuite d'électrons dans la chaine respiratoire au niveau des mitochondries.

Quel que soit leur procédé de formation, ces métabolites sont dangereux pour l'organisme en raison de leur haute réactivité et leur action affecte divers composants cellulaires, parmi lesquels un grand nombre de protéines structurales, des enzymes, des acides aminés, l'ADN et l'ARN, les glucides, ainsi que les lipides et les phospholipides.

A noter que dans le cas d'irradiations aux UV, outre la génération d'espèces réactives, il peut y avoir en plus un dommage physique direct sur les composants cellulaires. Ainsi, les UV sont connus pour induire des dimères de thymine qui peuvent engendrer des mutations, voire des cassures de l'ADN.

Dans une situation de production excessive de métabolites réactifs, l'organisme est confronté à un processus dit de « stress oxydatif » ou « stress oxydant ».

Pour se défendre contre les dommages causés par ces métabolites, l'organisme a développé des systèmes de défense contre l'oxydation pour diminuer la réactivité de ces substances, voire pour les neutraliser. Ces systèmes peuvent être de nature protéique, comme l'albumine sérique (HSA), la superoxyde dismutase (SOD), la catalase, les peroxydases ou des petits peptides, ou de nature non protéique, tels que l'ubiquinol, les anthocyanines, les flavonoïdes, les vitamines liposolubles (à titre d'exemple les vitamines A et E) et les vitamines hydrosolubles (à titre d'exemple la vitamine C). Les cellules possèdent également des systèmes de réparation et de dégradation des molécules oxydées.

Ainsi, pour lutter contre le stress oxydatif, il est conseillé d'adopter un régime riche en substances naturellement anti-oxydantes et de prendre des médicaments ou compléments alimentaires à base notamment de vitamines capables de neutraliser les espèces réactives. Dans le domaine cosmétique, de nombreux produits intègrent des agents antioxydants permettant notamment de lutter contre le vieillissement prématuré lié au stress oxydatif.

Par conséquent, la recherche de nouvelles molécules ou de nouveaux extraits présentant une activité anti-oxydante, à intégrer notamment dans des compléments alimentaires ou des compositions cosmétiques, est depuis de nombreuses années un enjeu majeur.

### Description de l'invention

La présente demande rapporte l'identification d'un extrait issu d'une bactérie extrêmophile, présentant une activité anti-oxydante d'intérêt. Outre un effet direct sur les radicaux libres, la présente demande rapporte un effet particulier d'un tel extrait sur les dommages causés par les UV voire par la lumière visible, et plus généralement un effet protecteur, voire stabilisant sur les protéines, ainsi qu'une synergie avec des antioxydants connus.

Ainsi, la présente demande illustre une composition cosmétique ou pharmaceutique comprenant un extrait de la bactérie *Arthrobacter agilis*, plus précisément un extrait contenant des caroténoïdes obtenu à partir de cette bactérie, et un agent antioxydant choisi dans le groupe de la vitamine E ou l'un de ses dérivés.

Le document KR 2012/0068367 propose une composition cosmétique comprenant le surnageant de culture, riche en exopolysaccharides, issu de la fermentation à basse température de microorganismes psychrophiles, notamment *Arthrobacter agilis.*

Le document JP H07 132096 décrit un protocole de préparation de caroténoïde de type bactériorubérine obtenue à partir de microorganismes de type *Halobacterium.*

Le document SAITO et al. (RADIATION PHYSICS AND CHEMISTRY, vol. 50, no. 3, 1997, pages 267-269) quant à lui, étudie l'effet de la bactériorubérine extraite de *Rubrobacter radiotolerans* sur la thymine exposée à des irradiations au cobalt. Cette étude rapporte un effet protecteur de la bactériorubérine sur la thymine vis-à-vis des radicaux OH.

Sont déjà disponibles des compositions cosmétiques anti-âge (« M.D. Formulated Age Antidote Day Cream », MINTEL Database accession no. 10194702 et « Shining Star Eye Cream », MINTEL Database accession no. 1305663) ou alimentaire (« Dietary Supplément », MINTEL Database accession no. 1230777) comprenant une multitude d'ingrédients dont des caroténoïdes d'origine différente ou non identifiée et de la vitamine E ou ses dérivés.

La bactérie *Arthrobacter agilis* a été par exemple décrite par Koch et al. (Int J Syst Bacteriol. 1995, 45(4):837-9) et peut être aisément identifiée, notamment grâce à la séquence de son ARN 16S accessible dans les bases de données sous le numéro X80748 et correspondant à la séquence SEQ ID NO : 1 suivante :

Ainsi et selon un mode de réalisation particulier, la souche bactérienne mise en œuvre possède un ARN 16S codé par une séquence présentant plus de 90%, voire plus de 95%, voire 99%, voire même 100% d'identité avec la séquence SEQ ID NO : 1, par exemple de séquence partielle SEQ ID NO : 2.

Selon l'invention, un tel extrait contient des caroténoïdes, avantageusement est riche en caroténoïdes.

Dans le cadre de l'invention, on entend par « caroténoïdes » des pigments liposolubles caractérisés par leur couleur pouvant aller du rouge au jaune et leur spectre d'absorption. Ils appartiennent à la famille chimique des terpénoïdes, à structure aliphatique ou alicyclique polyinsaturée.

Comme mentionné, un extrait selon l'invention contient des caroténoïdes, qui peuvent aussi bien être membranaires que cytoplasmiques.

De manière avantageuse et comme par exemple mis en évidence par une analyse HPLC, un extrait objet de la présente invention est riche en caroténoïdes. Les caroténoïdes présentent une zone d'absorption maximale entre 400 et 600 nm, plus précisément entre 450 et 550 nm, et sont identifiables par leur spectre d'absorption à « 3 doigts » à une longueur d'onde proche de 490 nm. Encore plus avantageusement, les caroténoïdes représentent plus de 50%, voire 60%, 70%, 80% ou même 90% en quantité de l'extrait. Préférablement, l'extrait est dépourvu de protéines et/ou d'ADN et/ou de glucides.

Selon un mode de réalisation privilégié, l'extrait selon l'invention contient différentes formes de caroténoïdes, avantageusement 6 formes principales, y compris différents isomères ou formes glycosylées.

Un extrait selon l'invention est avantageusement obtenu par culture d'*Arthrobacter agilis* dans les conditions suivantes :
Le milieu de culture utilisé est typiquement un milieu LB sans sel, à savoir contenant 1% en poids de tryptone et 0,5% en poids d'extrait de levure. Alternativement, des milieux R2A (Koch et al., 1995, Int J Syst Bacteriol. 45(4):837-9) et Bacto Marine 2216 (Fong et al., 2001, Appl Microbiol Biotechnol. 56(5-6):750-6) peuvent être envisagés.

Les cellules sont avantageusement cultivées à une température comprise entre 20 et 30°C, par exemple 25°C.

De manière adaptée, la croissance se fait dans des conditions d'aérobiose, préférentiellement sous aération par bulle d'air ou d'oxygène.

L'extrait selon l'invention est avantageusement obtenu à partir de cellules en phase stationnaire, soit typiquement après une croissance de 3 jours dans les conditions décrites ci-dessus.

Pour l'obtention de l'extrait, les cellules bactériennes sont soumises à un protocole d'extraction/purification permettant d'isoler et de purifier les caroténoïdes. En raison du caractère liposoluble des caroténoïdes, l'obtention de l'extrait objet de l'invention repose avantageusement sur l'isolation d'une phase apolaire.

Dans une première étape, il s'agit d'extraire des composants membranaires et cytoplasmiques, avantageusement à l'exception des lipides, des protéines et de l'ADN. De manière adaptée, un solvant polaire est mis en œuvre permettant de « casser » les membranes et de solubiliser un très grand nombre de molécules hydrophiles et certaines lipophiles.

Ainsi et à titre d'exemple, le culot bactérien peut être extrait à l'aide d'acétone, de méthanol ou d'un mélange acétone/méthanol (par exemple 1/5 en volume).

Dans une seconde étape, l'ajout d'un solvant apolaire, avantageusement de l'hexane, et d'une solution aqueuse saturée en sels, avantageusement une solution de NaCl saturée, permet l'obtention d'un mélange biphasique, à savoir une phase aqueuse d'un côté et la phase apolaire de l'autre correspondant à l'extrait d'intérêt dans le cadre de l'invention. De manière avantageuse, la phase aqueuse peut être lavée, éventuellement plusieurs fois, à l'aide du solvant apolaire.

Les phases apolaires sont alors réunies et évaporées sous vide. L'extrait ainsi obtenu peut être stocké au froid, en particulier congelé.

Il ressort clairement de ce procédé qu'il s'agit d'un extrait bactérien et non d'un surnageant de culture.

Selon un premier mode de réalisation, l'extrait selon l'invention est utilisé sous forme sèche, en tant que poudre. Alternativement, le culot peut être repris dans toute phase lipophile adaptée. Dans le cadre de l'application cosmétique selon l'invention, l'extrait est avantageusement repris dans une huile, du propanediol ou une micro-émulsion.

La concentration en caroténoïdes de la composition reconstituée à partir de l'extrait séché peut être aisément mesurée, par exemple par mesure de l'absorbance à 512 nm dans le DMSO ou à 502 nm dans le propanediol.

A noter que les différentes étapes d'extraction et de purification sont avantageusement réalisées à l'abri de la lumière, dans l'obscurité ou dans la pénombre, afin d'éviter l'éventuelle dégradation de l'extrait.

En raison des propriétés remarquables mises en évidence dans le cadre de la présente demande et exposées ci-dessous, un tel extrait peut notamment être utilisé dans une composition cosmétique, une composition pharmaceutique ou un complément alimentaire.

Selon un premier aspect, la présente invention concerne donc une composition cosmétique comprenant un extrait contenant des caroténoïdes de la bactérie *Arthrobacter agilis* et un agent antioxydant qui est la vitamine E. Selon un mode de réalisation privilégié, la composition cosmétique comprend un extrait riche en caroténoïdes de la bactérie *Arthrobacter agilis.*

La composition cosmétique selon l'invention est avantageusement topique, destinée à être appliquée sur la peau et éventuellement sur les cheveux. Elle peut contenir tout ingrédient ou excipient couramment utilisé en cosmétique. En d'autres termes, elle comprend au moins un excipient cosmétiquement acceptable, tous les excipients utilisés dans une telle composition devant être cosmétiquement acceptables.

A noter que le méthanol notamment n'est pas considéré comme un excipient cosmétiquement acceptable. Selon un mode de réalisation particulier, la composition cosmétique selon l'invention ne contient donc pas de méthanol.

Elle peut aussi bien se présenter sous forme de lotion, de crème, de gel, de spray, ...

Selon un mode de réalisation particulier, elle présente une couleur rosée.

Selon un autre aspect, la présente invention concerne donc une composition pharmaceutique comprenant un extrait contenant des caroténoïdes de la bactérie *Arthrobacter agilis* et un agent antioxydant qui est la vitamine E. Selon un mode de réalisation privilégié, la composition pharmaceutique comprend un extrait riche en caroténoïdes de la bactérie *Arthrobacter agilis.*

La composition pharmaceutique selon l'invention peut être notamment destinée à une administration topique ou orale. Elle peut contenir tout ingrédient ou excipient couramment utilisé en pharmacie. En d'autres termes, elle comprend au moins un excipient pharmaceutiquement acceptable, tous les excipients utilisés dans une telle composition devant être pharmaceutiquement acceptables.

A noter que le méthanol notamment n'est pas considéré comme un excipient pharmaceutiquement acceptable. Selon un mode de réalisation particulier, la composition pharmaceutique selon l'invention ne contient donc pas de méthanol.

Elle peut aussi bien se présenter sous forme de lotion, de crème, de gel, de spray, de solutions par exemple sous forme de gouttes, de gélules, de comprimés, ...

Une telle composition pharmaceutique peut par exemple être utilisée dans le domaine ophtalmique, notamment pour traiter certaines rétinopathies (DMLA, ..), dans le traitement des maladies chroniques du foie ou de l'hyperoxie qui suit l'ischémie-reperfusion. De manière plus générale sont visées les pathologies dans lesquelles un stress oxydant peut endommager irréversiblement les protéines.

Dans le même contexte, une composition selon la présente demande peut être un complément alimentaire.

Des compositions selon l'invention peuvent en outre contenir d'autres actifs, en plus de l'extrait objet de la présente invention.

Il peut en particulier s'agir d'autres agents antioxydants, regroupant différentes classes de molécules, en particulier des caroténoïdes, des thiols ou des phénols.

Un agent antioxydant, pour lequel un effet de synergie avec l'extrait selon l'invention a été mis en évidence, est la Vitamine E.

Des extraits végétaux, tels qu'un extrait de thé vert, un extrait de sarment de vigne, un extrait des feuilles d'arganier, un extrait de pamplemousse, un extrait de feuilles de murier et/ou un extrait de pomme, peuvent également être associés à un extrait selon l'invention.

Les concentrations en extrait et en actif, respectivement, sont déterminées au cas par cas par l'homme de métier, de manière à ce que la synergie entre les deux s'exprime pleinement. Ainsi et de manière adaptée, les concentrations de l'extrait et de l'actif supplémentaire doivent être optimisées pour l'effet anti-oxydant recherché.

De manière avantageuse, l'extrait sec représente de 0,00001 (10⁻⁵) à 0,1 (10⁻¹) %, avantageusement de 0,0001 (10⁻⁴) à 0,01 (10⁻²) % en poids de la composition selon l'invention.

En raison des propriétés remarquables mises en évidence dans le cadre de la présente demande, une telle composition peut en particulier être utilisée contre le vieillissement (effet anti-âge) ou pour la protection solaire (effet anti-UV).

Selon un autre aspect, la présente invention vise un procédé de traitement cosmétique, avantageusement pour lutter contre le stress oxydant, notamment le vieillissement cellulaire de la peau, consistant à appliquer sur la peau la composition cosmétique selon l'invention. Un stress oxydant peut également être causé par une exposition aux rayonnements (UV et/ou visible), contre lesquels la composition selon l'invention se montre particulièrement efficace.

Une première utilisation possible d'une composition selon l'invention est celui d'un agent anti-radicalaire, notamment vis-à-vis des radicaux libres suivants : le radical superoxyde O₂·⁻ ; le peroxyde d'hydrogène H₂O₂ ; l'ion hypochlorite ClO⁻ ; le radical hydroxyle OH· ; les radicaux peroxyde (ROO·) ou alkoxyle (RO·) où R est une chaîne carbonée ; les radicaux dérivés d'acides gras insaturés ; le peroxynitrite ONOO· ; le monoxyde d'azote NO· ; le dioxygène singulet ¹O₂.

Une composition selon l'invention peut également être utilisée comme agent anti-UV, aussi bien contre les UV-A (400-315nm), les UV-B (315-280nm) et/ou les UV-C (280-153nm), notamment contre les UV-C. De manière remarquable, il a été montré qu'une composition selon l'invention exerçait un effet anti-UV via plusieurs mécanismes d'action :
- un effet de neutralisation des singulets de l'oxygène (¹O₂) générés par les UV ;
- un effet « écran », « filtre » ou « bouclier », par absorption directe des UV ;
- un effet antioxydant plus classique, à savoir la neutralisation des radicaux libres générés par les singulets de l'oxygène (¹O₂).

Comme déjà dit, une composition selon l'invention présente également un intérêt dans la protection contre la lumière visible, en particulier vis-à-vis de la lumière bleu-violet (longueur d'onde typiquement comprise entre 400 et 500 nm).

Par ailleurs, une composition selon l'invention exerce un effet protecteur sur les protéines. Ainsi, une telle composition est susceptible de stabiliser et de protéger des protéines.

Comme démontré dans la présente demande, cette protection du protéome s'exerce notamment vis-à-vis de la carbonylation. De manière plus générale, la présente demande démontre le potentiel protecteur d'un extrait riche en caroténoïdes sur le protéome.

Ainsi et selon un autre aspect, la présente demande décrit l'utilisation des caroténoïdes pour la protection du protéome.

### Exemples de réalisation

La manière dont l'invention peut être réalisée et les avantages qui en découlent, ressortiront mieux des exemples de réalisation qui suivent, donnés à titre indicatif et non limitatif, à l'appui des figures annexées.
La figure 1 correspond à une image en microscopie inverse (grossissement x100) de la souche SB5.
La figure 2 représente le spectre HPLC de l'extrait SBE selon l'invention, ainsi que le spectre d'absorbance pour chacun des 6 pics principaux (notés 1 à 6).
La figure 3 représente le pouvoir antioxydant mesuré à l'aide du test ABTS de l'extrait selon l'invention (SBE) par rapport à d'autres agents antioxydants connus, exprimé en équivalent Trolox.
La figure 4 représente le pouvoir protecteur des protéines, vis-à-vis des radicaux libres, de l'extrait selon l'invention (SBE) par rapport à d'autres agents antioxydants, exprimé en équivalent Trolox (A), et compare ce pouvoir protecteur à celui des fractions correspondant aux 6 pics observés en HPLC (B).
La figure 5 représente le pouvoir protecteur des protéines, vis-à-vis des UV, de l'extrait selon l'invention (SBE) par rapport à d'autres agents antioxydants, exprimé en équivalent Trolox.
La figure 6 représente le suivi de l'activité de la phosphatase alcaline (AP), en fonction de la concentration de l'extrait selon l'invention (SBE).
La figure 7 représente le pouvoir protecteur des protéines, vis-à-vis des radicaux libres, de l'extrait selon l'invention (SBE) mélangé à d'autres agents antioxydants, exprimé en équivalent Trolox (B).
La figure 8 représente le pouvoir protecteur des cellules humaines (kératinocytes) et de leur ADN, vis-à-vis des radicaux libres et des UV, de l'extrait selon l'invention (SBE) par rapport à l'acétate de tocophérol via le test des comètes.
La figure 9 représente la mesure de la carbonylation des protéines évaluant le pouvoir protecteur des cellules humaines (kératinocytes) et de leurs protéines, vis-à-vis d'une irradiation UV/visible, de l'extrait selon l'invention (SBE) par rapport à l'acétate de tocophérol.

### 1/ Isolation et caractérisation de la souche SB5 :

Une bactérie (SB5) a été isolée dans la neige tombante et sélectionnée pour sa résistance aux UVC et sa capacité à croitre rapidement. Elle a été caractérisée comme appartenant aux Arthrobacters, bactéries grams positives dont beaucoup d'espèces sont extrêmophiles ou extrêmotolérantes.

Plus précisément, la bactérie isolée est une souche d'*Arthrobacter agilis. A. agilis* est une bactérie coccoïde gram positive, non-sporulante et non pathogène, de 0,8 à 1,2 µm de diamètre et pouvant posséder de 0 à 3 flagelles (Koch et al. (1995) Int J Syst Bacteriol. 45(4):837-9), isolée pour la première fois en 1889 (Ali-Cohen (1889) Zentralbl. Bakteriol. Parasitenkd. Infektionskr. Hyg. Abt. 1 Orig. 6:33-36).

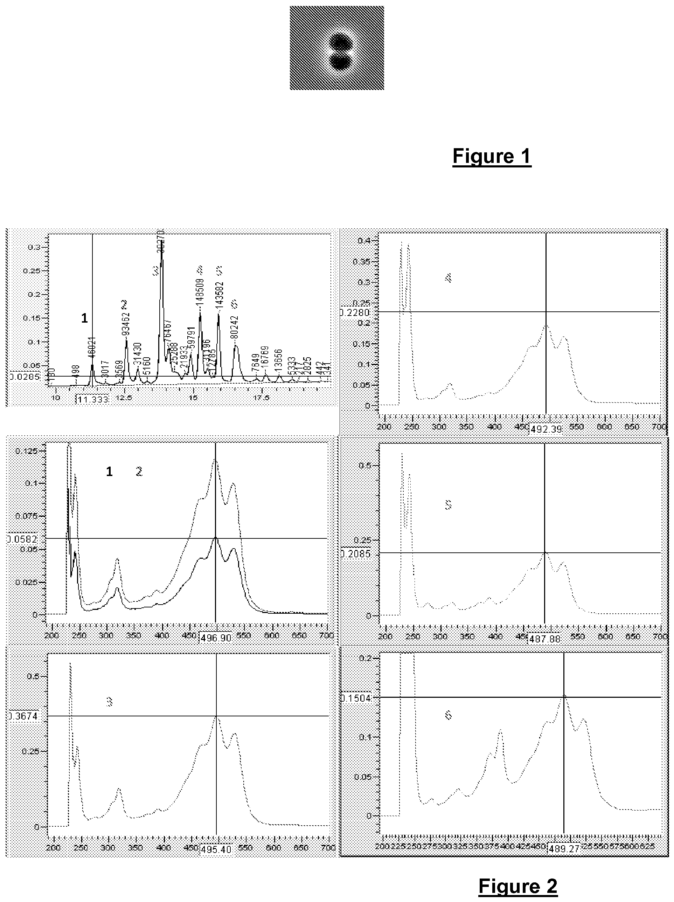

La figure 1 montre une image en microscopie inverse de la souche isolée, conforme à la description de la bactérie *Arthrobacter agilis.*

Par ailleurs, l'appartenance de la souche SB5 à l'espèce *Arthrobacter agilis* a été confirmée par séquençage partiel de son ARN 16S. La séquence correspondante (SEQ ID NO: 2) est comme suit, la base N indiquant qu'il n'a pas pu être déterminé si le nucléotide correspondait à A, C, G ou T :

Cette séquence présente effectivement plus de 99,2% d'identité avec la séquence de référence SEQ ID NO : 1, confirmant l'appartenance de cette souche à l'espèce *Arthrobacter agilis.*

### 2/ Préparation de l'extrait SBE :

### 2-1. Culture de la souche SB5 :

La souche est cultivée dans du milieu LB sans sel à une température de 25°C, en aérobie et sous forte agitation, jusqu'à ce que la phase stationnaire soit atteinte, soit environ après 3 jours.

Les cellules sont alors collectées par centrifugation, typiquement à une vitesse de 5000 rpm pendant environ 15 minutes. Le culot est ensuite séché par stockage à l'obscurité à 4°C.

### 2-2. Isolation de l'extrait SBE :

Le culot est repris dans 6 ml d'acétone par gramme de culot, éventuellement en présence de méthanol, par exemple dans un mélange méthanol/acétone (5/1). Cette étape d'extraction a lieu pendant plusieurs heures, typiquement 18 heures, à 4°C et à l'obscurité.

La suspension dans l'acétone, ou dans le mélange acétone/méthanol, est complétée par ajout d'hexane, avantageusement à la moitié du volume d'acétone. Une solution saturée en NaCl (chlorure de sodium) est alors ajoutée jusqu'à séparation du mélange biphasique, avec une phase aqueuse d'un côté et une phase apolaire de l'autre. De manière avantageuse, la phase aqueuse est à nouveau extraite à l'aide d'hexane et les phases hexaniques sont réunies.

La phase apolaire ou hexanique est évaporée, avantageusement sous vide et à 25°C.

L'extrait SBE peut être conservé en l'état à -20°C ou peut être repris en solution, par exemple dans du DMSO (diméthylsulfoxyde) ou du THF (tétrahydrofurane) ou du propanediol pour être testé sur cellules vivantes, et est très soluble dans le dichlorométhane et l'acétone.

La concentration en caroténoïdes est déterminée par mesure de l'absorbance à 512 nm dans le DMSO ou à 502 nm dans le propanediol.

Le spectre HPLC de l'extrait, ainsi que le spectre d'absorbance pour chacun des 6 pics principaux, sont représentés à la Figure 2. La comparaison de ces spectres avec les données de la littérature (Fong et al., Appl Microbiol Biotechnol (2001) 56 : 750-756) montre que l'extrait est constitué très majoritairement de caroténoïdes, avec un spectre caractéristique d'absorption à 3 « doigts » vers 490 nm. Comme indiqué dans Fong et al., (Appl Microbiol Biotechnol (2001) 56 : 750-756), le spectre d'absorption révèle également la présence de formes glycosylées et de différents isomères de ces caroténoïdes.

### 3/ Activités de l'extrait SBE :

### 3-1. Test ABTS :

Comme mentionné ci-dessus, ce test classiquement utilisé dans l'industrie cosmétique et agro-alimentaire permet d'établir, de façon purement chimique, le « pouvoir antioxydant total » d'un extrait. Bien que ce test soit incomplet car il ne mesure en fait l'activité antioxydante de l'extrait que face à un type particulier de radical, il présente l'avantage, par sa normalisation avec le Trolox, de donner des résultats qui sont comparables avec des milliers d'autres molécules testées de cette manière.

Ce test a été mis en œuvre comme décrit dans Re et al., Free Radic. Biol. Med. (1999) 26(9-10) : 1231-7.

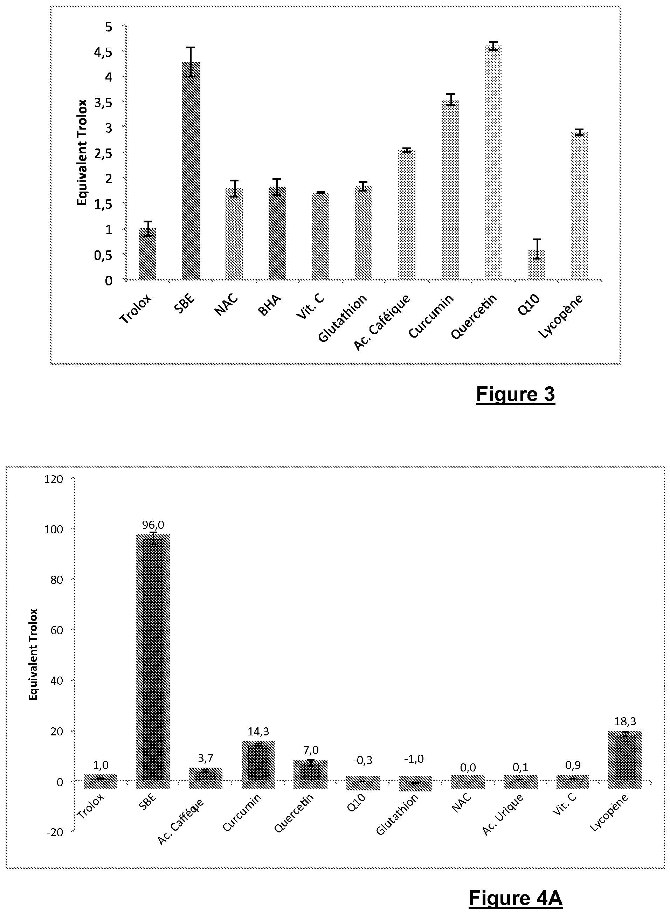

La figure 3 révèle que l'extrait selon l'invention (SBE) montre un pouvoir antioxydant plus de 4 fois supérieur à celui de l'antioxydant de référence, le Trolox.

Dans le cadre de l'invention, un équivalent Trolox correspondant au nombre de moles du produit testé permettant d'obtenir un effet équivalent à celui d'1 mole de Trolox. L'extrait selon l'invention a été quantifié grâce à l'utilisation des caroténoïdes comme marqueur de l'extrait. Ainsi, 1 mole de l'extrait SBE correspond à 1 mole de caroténoïdes, tel que mesuré par absorbance à la longueur d'onde correspondant au maximum d'absorption et déduit du coefficient d'extinction molaire des caroténoïdes.

### 3-2. Protection des protéines contre les radicaux libres :

Ce test consiste à mesurer à 405 nm l'activité de la phosphatase alcaline (AP) en présence de radicaux libres, notamment hydroxyles (OH·), et d'un composé ou extrait d'intérêt, afin d'évaluer le pouvoir protecteur vis-à-vis des protéines dudit composé ou extrait.

### Matériel et méthodes :

### Réactifs :

Tampon : poudre de « Tris Buffered Saline » (Sigma ; Référence produit : T6664-10PAK) ;
Enzyme : Phosphatase alcaline 10KU à 125U/µL dans une solution de glycérol tamponnée (Sigma ; Référence produit : P0114) ;
Agent oxydant :
   1/ Peroxyde d'hydrogène à 30% ou 9M (Sigma ; Référence produit : H1009-5ML) : solution B1 ;
   2/ Sulfate de fer II heptahydrate (FeSO4, 7H₂O) 0,1M (Fluka ; Référence produit: 44970) : solution B2 ;
Substrat : solution de 4-nitrophényl phosphate (« Alkaline Phosphatase yellow substrate » ; Sigma ; Référence produit : P7998-100ML).
*Kit :*
   - Plaque 96 puits ;
   - Tampon : 20 ml d'une solution TBS 0,05M à pH 8 ;
   - Solution A (enzyme) : 10 µl de la solution mère diluée au 1/1000 (1,25U soit 0,125U/µL) dans du tampon ;
   - 100 µl de solution B1 et 100 µl de solution B2 ou solution B correspondant au mélange des solutions B1 et B2 à une dilution adaptée dans du tampon (par exemple 3 ml de solution B à 90 mM, obtenue à partir de 30 µl de solution B1 + 30 µl de solution B2 soit une dilution au 1/100) ;
   - Solution C (substrat) : 5 ml de la solution commerciale.
Protocole :
   - Diluer la solution A dans du tampon à une dilution adaptée ;
   - Déposer, dans chaque puits, 10 µl de la solution A diluée de sorte à avoir une quantité d'enzyme comprise entre 0,005 et 0,05 U ;
   - Ajouter 10 µl du produit ou de l'extrait à tester, éventuellement dilué dans du tampon, dans chaque puits. Les différents puits peuvent servir à tester une gamme de dilution ;
   - Ajouter 30 µl de la solution B dans chaque puits. La concentration de la solution B est ajustée pour obtenir une inactivation de 90% de la quantité d'enzyme en présence. La concentration finale de l'agent oxydant correspondant à 30% de la concentration de la solution B ;
   - Incuber 15 minutes à 37°C sans agitation ;
   - Ajouter 50 µl de la solution C. Le volume réactionnel est donc égal à 100 µl ;
   - Placer la plaque à 37°C sous agitation ;
   - Lire l'absorbance à 405 nm pendant 20 minutes (cinétique) ou après 5 minutes de réaction (ponctuelle).

### Résultats :

La figure 4A révèle que l'extrait selon l'invention (SBE) présente un pouvoir protecteur des protéines, face aux radicaux libres, environ 100 fois plus élevé que celui du Trolox, et plus de 6 fois supérieur à tous les antioxydants testés. La différence entre ce résultat et le potentiel antioxydant mesuré par ABTS démontre que la protection des protéines n'est pas uniquement liée au potentiel antioxydant de SBE.

Par ailleurs, cette même technique a été utilisée pour comparer l'effet des 6 fractions (notées SB1 à SB6) correspondant aux 6 pics principaux repérés par l'analyse HPLC à celui de l'extrait SBE total.

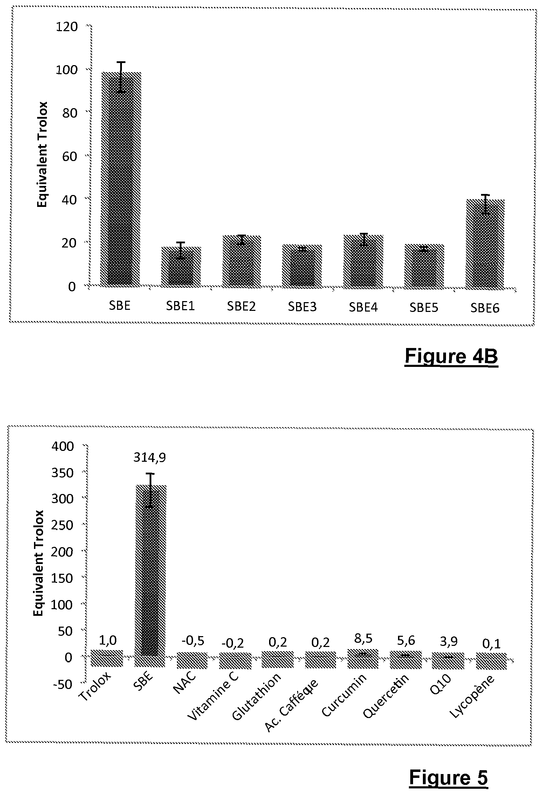

Il ressort de la figure 4B que l'extrait SBE a un pouvoir protecteur des protéines face aux radicaux libres de 2 à 4 fois plus élevé que celui de chacun des pics qui le compose et démontre un effet de synergie entre les différentes fractions de l'extrait.

### 3-3. Protection des protéines contre les UV :

Ce test est similaire à celui décrit dans la section précédente. Il consiste à mesurer à 405 nm l'activité de la phosphatase alcaline (AP), irradiée à l'aide d'UV-C (254 nm), en présence d'un composé ou extrait d'intérêt, afin d'évaluer le pouvoir protecteur vis-à-vis des protéines dudit composé ou extrait.

### Matériel et méthodes :

La phosphatase alcaline a été soumise à une irradiation UV à 254 nm correspondant aux UV-C. Sous agitation constante, des doses d'UV permettant une inactivation de 90% de l'enzyme ont été appliquées sur un milieu réactionnel et dans des conditions similaires à ceux mentionnés ci-dessus. A noter que le volume occupé par l'agent oxydant est ici remplacé par du tampon. En pratique, il été utilisé une lampe d'une puissance égale à 0,0365J/cm²/min, avec un temps d'exposition de 1 ou 2 heures.

### Résultats :

La figure 5 révèle que l'extrait selon l'invention (SBE) présente un pouvoir protecteur des protéines, vis-à-vis des UV, plus de 300 fois plus élevé que celui du Trolox et près de 40 fois supérieur à tous les antioxydants testés.

Ce très fort effet protecteur de l'extrait selon l'invention pourrait s'expliquer par plusieurs effets conjugués de l'extrait en présence :
- un effet de neutralisation des singulets de l'oxygène (¹O₂) générés par les UV ;
- un effet « écran », « filtre » ou « bouclier », par absorption directe des UV ;
- un effet antioxydant plus classique, à savoir la neutralisation des radicaux libres générés par les singulets de l'oxygène (¹O₂).

### 3-4. Protection des protéines :

L'activité de la phosphatase alcaline a été mesurée en l'absence de tout stress oxydant mais en présence de concentrations croissantes de l'extrait selon l'invention (SBE).

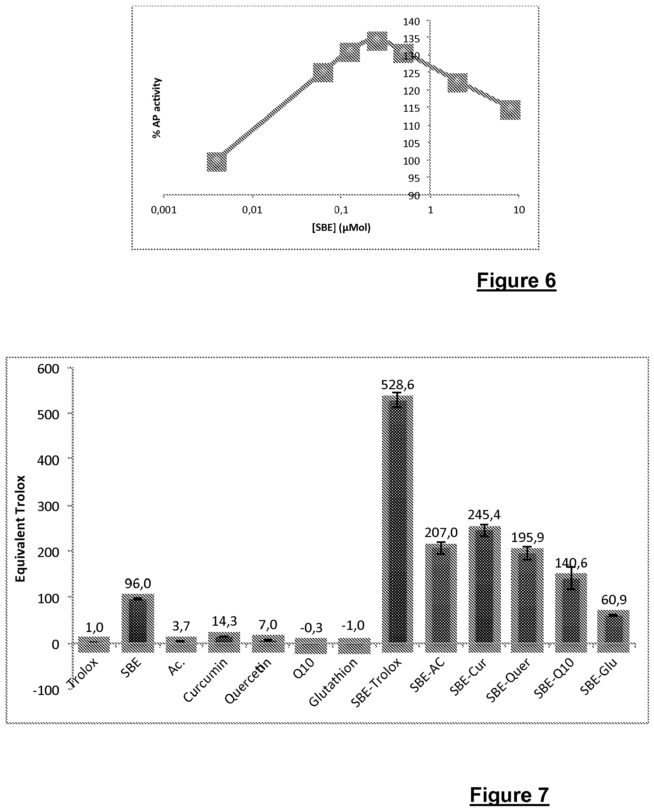

La figure 6 révèle que l'activité de l'AP est augmentée en présence de l'extrait selon l'invention (SBE) à très faibles doses. Cette augmentation pourrait être due à la protection de l'enzyme pendant les étapes d'incubation et d'agitation. Il est également envisageable que certaines oxydations subies par l'enzyme stockée à -20°C soient réduites par l'extrait SBE, ce qui pourrait augmenter son activité par « rajeunissement ».

Sur la base de la figure 6, une concentration adaptée en extrait selon l'invention (SBE) se situe entre 0,001 et 100 µM, avantageusement entre 0,1 et 1 µM. Il s'agit toutefois de concentrations à atteindre dans les cellules cibles de la peau. Ainsi, la concentration dans les compositions cosmétiques doit être bien supérieure pour prendre en compte les pertes liées à la pénétration cutanée.

Une interaction entre l'enzyme et l'extrait pourrait être à l'origine de cette action « boostante ». Par extension, l'extrait selon l'invention est susceptible de protéger les protéines présentes dans l'organisme, notamment celles impliquées directement dans la protection de l'organisme contre le stress oxydatif ou dans la réparation des dommages oxydatifs.

### 3-5. Synergie avec d'autres antioxydants :

Afin d'identifier des antioxydants susceptibles d'augmenter l'efficacité de la protection des protéines par l'extrait selon l'invention (SBE), le test décrit à la section 3-2 a été mis en œuvre en mélangeant l'extrait selon l'invention (SBE) et une dizaine d'antioxydants connus, pris individuellement. L'intérêt est d'identifier des molécules avec lesquelles l'extrait selon l'invention (SBE) cumule les effets antioxydants, voire de mettre en évidence des synergies allant au-delà des effets additifs. Un autre intérêt de tels mélanges est la possible stabilisation de l'extrait selon l'invention (SBE).

La figure 7 montre qu'en présence de l'ensemble des antioxydants testés, et particulièrement en présence de Trolox, l'extrait selon l'invention (SBE) présente une forte synergie, qui augmente significativement (d'un facteur supérieur à 5 avec le Trolox) son pouvoir protecteur des protéines vis-à-vis des radicaux libres.

### 3-6. Protection des cellules Humaines :

### A/ Test des comètes :

Afin de confirmer la protection par l'extrait selon l'invention (SBE) exercée sur les cellules humaines, des mesures de la protection de cultures primaires de kératinocytes et de leur ADN face à un stress oxydant induit par la lumière UV/visible a été mis en œuvre grâce au test dit des comètes, tel que décrit par Ostling, O., and K. J. Johanson. (Biochemical and biophysical research communications 123.1 (1984): 291-298) et Singh, Narendra P., et al. (Experimental cell research 175.1 (1988): 184-191). L'intérêt est de montrer l'activité de protection de l'extrait selon l'invention (SBE) dans un modèle cellulaire et de le comparer à un antioxydant de référence, l'acétate de tocophérol.

### Matériel et méthodes :

Les propriétés antioxydantes du SBE et de l'acétate de tocophérol (Ac-Toc) contre des rayonnements UVB/UVA/VISIBLE (Irradié : 290 nm - 800 nm) ont été évaluées par le test des comètes (version alcaline) sur des primo-cultures de kératinocytes humains normaux.

L'évaluation des propriétés antioxydantes a été effectuée aux concentrations de 500 nM pour SBE et 50 µM pour Ac-Toc. Les deux produits ont été mis en solution dans du tétrahydrofurane à une concentration finale de 1%. Les propriétés antioxydantes ont été définies comme la capacité à diminuer le nombre de cassures simple brin de l'ADN des cellules qui est produit après une irradiation UVB/UVA/Visible (290-800 nm) et ces propriétés ont été mesurées après un contact avec les produits de 120 min à 37°C. L'irradiation a été délivrée par un simulateur solaire Suntest CPS+ (Atlas Material Testing Technology BV, Moussy le Boeuf, France). La dose totale d'irradiation était de 12.0 J/cm2 pour une période de 2.7 min (puissance nominale de la lampe 750 W/m2). Les contrôles négatifs comprenaient des kératinocytes traités par THF (1%) et non irradiés, par Ac-Toc (50 µM) et SBE (500 nM) dans THF 1% et non irradiés.

### Résultats :

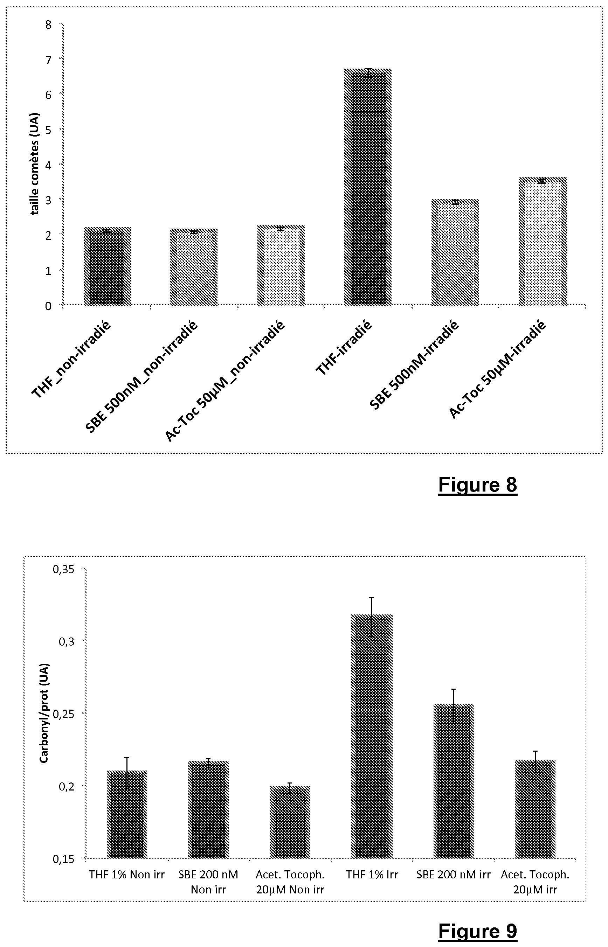

La figure 8 montre que l'extrait selon l'invention (SBE) présente un fort potentiel protecteur des cellules face à un stress oxydant et qu'il permet de protéger l'ADN des cellules mieux que l'acétate de Tocophérol, tout en étant 100 fois moins concentré. Son pouvoir protecteur in vitro est donc plus de 100 fois plus important que celui de l'acétate de tocophérol.

### B/ Test de carbonylation :

En suivant le même protocole d'irradiation UV/visible que celui utilisé ci-dessus pour le test des comètes, le taux de carbonylation des kératinocytes en présence de SBE et acétate de tocophérol a été mesuré.

### Matériel et méthodes :

### 1- Culture des kératinocytes et irradiation

- Kératinocytes humains normaux (KHN) (T6)-10⁶ cellules/condition
- Conditions :
   ▪ Témoin solvant THF 1%
   ▪ SBE 200 nM
   ▪ Acétate de tocophérol 20 µM
- Chaque condition est réalisée en triplicat.
- Mise en contact des KHN dans les différentes conditions expérimentales pendant 2h.
- Irradiation UVB UVA Visible à 120kJ/m² des cellules placées dans PBS par un simulateur solaire Suntest CPS+ (Atlas Material testing technology) à 4°C. Les contrôles négatifs sont maintenus à 4°C pendant la même durée que l'irradiation.
- Trypsination des cellules post irradiation. Rinçage du PBS glacé. Elimination du PBS et congélation immédiate des culots cellulaires à -80°C.

### 2- Mesure de la carbonylation

- Les protéines sont extraites après lyse des cellules et leur concentration est mesurée par test de Bradford.
- Les protéines sont dérivées par 2,4-dinitrophénylhydrazine (DNPH) qui se fixe aux groupements carbonyle.
- Le taux de carbonylation est ensuite mesuré sur plaques ELISA (OxyELISA™ Oxidized Protein Quantitation Kit, Millipore) à 450 nm.

### Résultats :

La figure 9 montre que le taux de carbonylation des protéines des kératinocytes augmente significativement après irradiation UV/visible. Cette augmentation est significativement diminuée (P-value < 0,01) par l'ajout de l'extrait SBE ou d'acétate de tocophérol qui protège les protéines contre l'oxydation. Cette protection est similaire (P-value > 0,01) pour SBE et acétate de tocophérol bien que l'extrait SBE soit 100 fois moins concentré.

Bien que légèrement moins fort, le potentiel de protection du protéome par le SBE est du même ordre que celui observé via le test des comètes et de l'ordre de 100 fois celui de l'acétate de tocophérol, ce qui correspond aux résultats obtenus ci-dessus basés sur la mesure de l'activité de la phosphatase alcaline (PA).

La protection des cellules observée via le test des comètes semble donc principalement portée par la capacité de SBE à protéger le protéome.

### 4/ Composition cosmétique contenant l'extrait SBE

Les compositions cosmétiques comprennent typiquement de 0,0001 à 0,01 % en poids de l'extrait (sec) selon l'invention.

Une crème peut présenter la composition suivante (en % massique), l'actif constitué par l'extrait selon l'invention étant avantageusement intégré dans l'isononyl isononanoate.

| **Nom INCI** | **% pondéral** |
|---|---|
| Aqua | 78,290000 |
| Xanthan Gum | 0,100000 |
| Ammonium Acryloyldimethyltaurate/VP Copolymer & Aqua | 0,800000 |
| Glycerin | 5,000000 |
| Butylene Glycol | 2,000000 |
| Phenoxyethanol | 0,350000 |
| Disodium EDTA | 0,200000 |
| Chlorphenesin | 0,260000 |
| Steareth-21 | 1,160000 |
| Steareth-2 | 1,840000 |
| Dimethicone | 2,000000 |
| Glyceryl Dibehenate & Tribehenin & Glyceryl Behenate | 0,500000 |
| Isononyl Isononanoate | 5,000000 |
| Acacia Decurrens/Jojoba/Sunflower Seed Wax/Polyglyceryl-3 Esters | 1,000000 |
| Cetyl Alcohol | 1,500000 |

De manière caractéristique, ces compositions peuvent présenter une couleur allant du rosé clair au rouge, en raison de la couleur de l'extrait selon l'invention.

Ces exemples de réalisation démontrent que le choix particulier de la bactérie *Arthrobacter agilis* comme source de caroténoïdes (présentes dans de nombreuses espèces, notamment plantes algues, cyanobactéries, champignons, ..) présente des avantages inattendus dans le cadre des applications visées :
- Culture de la bactérie et préparation de l'extrait relativement aisés (contrairement à beaucoup d'organismes extrêmophiles, notamment halobacter);
- Synergie entre les différentes fractions de l'extrait ;
- Contribution des différentes isoformes à l'activité globale de l'extrait (contrairement par exemple à la seule déinoxanthine de *Deinococcus radiodurans*) ;
- Très forte activité anti-radicalaire et anti-UV par rapport aux antioxydants connus ;
- Synergie avec les antioxydants connus ;
- Effet protecteur encore jamais révélé vis-à-vis du protéome, qui va au-delà du potentiel antioxydant : Le potentiel antioxydant de l'extrait SBE est 4 fois supérieur à celui du Trolox, alors que sa capacité à protéger les protéines est 100 fois plus fort que celle du Trolox, et ce aussi bien dans le cadre de tests biochimiques que sur cultures cellulaires.

## Revendications

1. Composition cosmétique ou pharmaceutique comprenant :
- un extrait contenant des caroténoïdes de la bactérie *Arthrobacter agilis* ; et
- un agent antioxydant qui est la vitamine E.

2. Composition selon la revendication 1, ***caractérisée* en ce que** l'extrait est obtenu par culture de la bactérie dans un milieu constitué de 1 % en poids de tryptone et 0,5 % en poids d'extrait de levure.

3. Composition selon la revendication 1 ou 2, ***caractérisée* en ce que** l'extrait est obtenu à partir de cellules en phase stationnaire.

4. Composition selon l'une des revendications 1 à 3, ***caractérisée* en ce que** l'extrait correspond à la phase apolaire obtenue à partir du culot bactérien.

5. Composition selon la revendication 4, ***caractérisée* en ce que** le culot bactérien est extrait, avantageusement par de l'acétone et/ou du méthanol, et que la phase apolaire est obtenue par ajout d'un solvant apolaire, avantageusement de l'hexane, et d'une solution saturée en sels, avantageusement une solution de NaCl.

6. Composition selon l'une des revendications précédentes, ***caractérisée* en ce que** l'extrait représente de 0,0001 à 0,01 % en poids sec de la composition.

7. Composition selon l'une des revendications précédentes, ***caractérisée* en ce qu'**elle se présente sous forme de lotion, de crème, de gel, de spray, de solutions, de gélules ou de comprimés.

8. Procédé de traitement cosmétique, avantageusement pour lutter contre le stress oxydant, et notamment le vieillissement cellulaire de la peau, consistant à appliquer sur la peau la composition selon l'une des revendications 1 à 7.

9. Composition comprenant :
- un extrait contenant des caroténoïdes de la bactérie *Arthrobacter agilis* ; et
- un agent antioxydant qui est la vitamine E,
pour utilisation comme agent anti-UV.

10. Composition comprenant :
- un extrait contenant des caroténoïdes de la bactérie *Arthrobacter agilis* ; et
- un agent antioxydant qui est la vitamine E,
pour utilisation comme agent de protection contre la lumière visible, en particulier dans le domaine du bleu-violet.

11. Composition comprenant :
- un extrait contenant des caroténoïdes de la bactérie *Arthrobacter agilis* ; et
- un agent antioxydant qui est la vitamine E,
pour utilisation comme agent anti-radicalaire.

12. Composition comprenant :
- un extrait contenant des caroténoïdes de la bactérie *Arthrobacter agilis* ; et
- un agent antioxydant qui est la vitamine E,
pour utilisation comme agent protecteur des protéines.

## Patentansprüche

1. Kosmetische oder pharmazeutische Zusammensetzung, die enthält:
- einen Extrakt mit Karotinoiden des Bakteriums *Arthrobacter agilis;* und
- ein Antioxydationsmittel, bei dem es sich um Vitamin E handelt.

2. Zusammensetzung nach Anspruch 1, ***dadurch gekennzeichnet, dass*** der Extrakt durch Anzucht des Bakteriums in einem Medium, bestehend aus 1 Gewichts-% Trypton und 0,5 Gewichts-% Hefeextrakt hergestellt wird.

3. Zusammensetzung nach Anspruch 1 oder 2, ***dadurch gekennzeichnet, dass*** der Extrakt ausgehend von Zellen in der stationären Phase erhalten wird.

4. Zusammensetzung nach einem der Ansprüche 1 bis 3, ***dadurch gekennzeichnet, dass*** der Extrakt der apolaren Phase entspricht, die mittels der Bakterienprobe erhalten wird.

5. Zusammensetzung nach Anspruch 4, ***dadurch gekennzeichnet, dass*** die Bakterienprobe vorteilhafterweise mit Azeton und/ oder Methanol extrahiert wird und die apolare Phase durch Zugabe eines apolaren Lösungsmittels, vorteilhafterweise Hexan, und einer Salzlösung, vorteilhafterweise eine NaCl-Lösung, entsteht.

6. Zusammensetzung nach einem der vorangehenden Ansprüche, ***dadurch gekennzeichnet, dass*** der Extrakt 0,0001 bis 0,01 % Trockengewicht der Zusammensetzung bildet.

7. Zusammensetzung nach einem der vorangehenden Ansprüche, ***dadurch gekennzeichnet, dass*** sie die Form einer Lotion, einer Creme, eines Gels, eines Sprays, einer Lösung, einer Kapsel oder einer Tablette hat.

8. Verfahren zur kosmetischen Behandlung, vorteilhafterweise zur Bekämpfung von oxidativem Stress und insbesondere von der Zellalterung der Haut, das darin besteht, die Zusammensetzung nach einem der vorangehenden Ansprüche 1 bis 7 auf die Haut aufzutragen..

9. Zusammensetzung mit:
- einem Extrakt mit Karotinoiden des Bakteriums *Arthrobacter agilis;* und
- einem Antioxydationsmittel, bei dem es sich um Vitamin E handelt,
zum Einsatz als UV-Schutzmittel.

10. Zusammensetzung mit:
- einem Extrakt mit Karotinoiden des Bakteriums *Arthrobacter agilis;* und
- einem Antioxydationsmittel, bei dem es sich um Vitamin E handelt,
zum Einsatz als Schutz vor sichtbarem Licht, insbesondere im Bereich blau- violett.

11. Zusammensetzung mit:
- einem Extrakt mit Karotinoiden des Bakteriums *Arthrobacter agilis;* und
- einem Antioxydationsmittel, bei dem es sich um Vitamin E handelt,
zum Einsatz als Schutz gegen freie Radikale.

12. Zusammensetzung mit:
- einem Extrakt mit Karotinoiden des Bakteriums *Arthrobacter agilis;* und
- einem Antioxydationsmittel, bei dem es sich um Vitamin E handelt,
zum Einsatz als Protein-Schutzmittel.

## Claims

1. A cosmetic or pharmaceutical composition comprising:
- an extract containing carotenoids of the bacteria *Arthrobacter agilis*; and
- an antioxidant which is vitamin E.

2. The composition according to claim 1, ***characterized in that*** the extract is obtained by culturing the bacteria in a medium consisting of 1 % by weight of tryptone and 0.5% by weight of yeast extract.

3. The composition according to claim 1 or 2, ***characterized in that*** the extract is obtained from cells in the stationary phase.

4. The composition according to one of claims 1 to 3, ***characterized in that*** the extract corresponds to the apolar phase obtained from the bacterial pellet.

5. The composition according to claim 4, ***characterized in that the*** bacterial pellet is extracted, preferably using acetone and/or methanol, and the apolar phase is obtained by adding an apolar solvent, preferably hexane, and a saturated salt solution, preferably an NaCl solution.

6. The composition according to one of the preceding claims, ***characterized in that*** the extract represents from 0.0001 to 0.01% by dry weight of the composition.

7. The composition according to one of the preceding claims, ***characterized in that*** it is in the form of a lotion, cream, gel, spray, solutions, capsules or tablets.

8. A method of cosmetic treatment, preferably to fight against oxidative stress, and in particular cellular ageing of the skin, consisting of applying to the skin the composition according to one of claims 1 to 7.

9. A composition comprising:
- an extract containing carotenoids of the bacteria *Arthrobacter agilis*; and
- an antioxidant which is vitamin E,
for use as an anti-UV agent.

10. A composition comprising:
- an extract rich containing carotenoids of the bacteria *Arthrobacter agilis*; and
- an antioxidant which is vitamin E,
for use as an agent for protecting against visible light, in particular blue-violet light.

11. A composition comprising:
- an extract rich containing carotenoids of the bacteria *Arthrobacter agilis*; and
- an antioxidant which is vitamin E,
for use as a free-radical scavenger.

12. A composition comprising:
- an extract containing carotenoids of the bacteria *Arthrobacter agilis*; and
- an antioxidant which is vitamin E,
for use as a protective agent for proteins.


### SEQUENCE LISTING

<110> THOREL, Jean-Noël
<120> EXTRAIT D'ARTHROBACTER AGILIS POUR SON UTILISATION NOTAMMENT EN
   COSMETIQUE
<130> T131-B-35659 PCT
<150> FR 13.53200
   <151> 2013-04-09
<160> 2
<170> BiSSAP 1.2
<210> 1
   <211> 1482
   <212> DNA
   <213> Arthrobacter sp.
<220>
   <221> source
   <222> 1..1482
   <223> /mol_type="unassigned DNA" /organism="Arthrobacter sp."
<400> 1
   gatcctggct caggatgaac gctggcggcg tgcttaacac atgcaagtcg aacgatgaac      60
   ctcacttgtg gggggattag tggcgaacgg gtgagtaaca cgtgagtaac ctgcccttga     120
   ctctgggata agcctgggaa accgggtcta atactggata cgaccttctg gcgcatgcca     180
   tgttggtgga aagcttttgt ggttttggat ggactcgcgg cctatcagct tgttggtggg     240
   gtaatggcct accaaggcga cgacgggtag ccggcctgag agggtgaccg gccacactgg     300
   gactgagaca cggcccagac tcctacggga ggcagcagtg gggaatattg cacaatgggc     360
   gcaagcctga tgcagcgacg ccgcgtgagg gatgaaggcc ttcgggttgt aaacctcttt     420
   cagtagggaa gaagcctgtc ttttgggtgg gtgacggtac ctgcagaaga agcgccggct     480
   aactacgtgc cagcagccgc ggtaatacgt agggcgcaag cgttatccgg aattattggg     540
   cgtaaagagc tcgtaggcgg tttgtcgcgt ctgccgtgaa agtccggggc ttaactccgg     600
   atctgcggtg ggtacgggca gactagagtg cagtagggga gactggaatt cctggtgtag     660
   cggtgaaatg cgcagatatc aggaggaaca ccgatggcga aggcaggtct ctgggctgta     720
   actgacgctg aggagcgaaa gcatggggag cgaacaggat tagataccct ggtagtccat     780
   gccgtaaacg ttgggcacta ggtgtggggg acattccacg ttttccgcgc cgtagctaac     840
   gcattaagtg ccccgcctgg ggagtacggc cgcaaggcta aaactcaaag gaattgacgg     900
   gggcccgcac aagcggcgga gcatgcggat taattcgatg caacgcgaag aaccttacca     960
   aggcttgaca tgaaccggaa tgatgcagag atgtgtcagc cacttgtggc cggtttacag    1020
   gtggtgcatg gttgtcgtca gctcgtgtcg tgagatgttg ggttaagtcc cgcaacgagc    1080
   gcaaccctcg ttccatgttg ccagcgggtt atgccgggga ctcatgggag actgccgggg    1140
   tcaactcgga ggaaggtggg gacgacgtca aatcatcatg ccccttatgt cttgggcttc    1200
   acgcatgcta caatggccgg tacaaagggt tgcgatactg tgaggtggag ctaatcccaa    1260
   aaagccggtc tcagttcgga ttgaggtctg caactcgacc tcatgaagtt ggagtcgcta    1320
   gtaatcgcag atcagcaacg ctgcggtgaa tacgttcccg ggccttgtac acaccgcccg    1380
   tcaagtcacg aaagttggta acacccgaag ccggtggcct aaccccttgt gggagggagc    1440
   cgtcgaaggt gggaccggcg attgggacta agtcgtaaca ag                       1482
<210> 2
   <211> 1400
   <212> DNA
   <213> Arthrobacter sp.
<220>
   <221> source
   <222> 1..1400
   <223> /mol_type="unassigned DNA" /organism="Arthrobacter sp."
<220>
   <221> misc_feature
   <222> 571,671,1358,1376,1390
   <223> /note="A ou C ou G ou T"
<400> 2
   acatgcaagt cgaacgatga acctcacttg tggggggatt agtggcgaac gggtgagtaa      60
   cacgtgagta acctgccctt gactctggga taagcctggg aaaccgggtc taatactgga    120
   tacgaccttc tggcgcatgc catgttggtg gaaagctttt gtggttttgg atggactcgc    180
   ggcctatcag cttgttggtg gggtaatggc ctaccaaggc gacgacgggt agccggcctg    240
   agagggtgac cggccacact gggactgaga cacggcccag actcctacgg gaggcagcag    300
   tggggaatat tgcacaatgg gcgcaagcct gatgcagcga cgccgcgtga gggatgaagg    360
   ccttcgggtt gtaaacctct ttcagtaggg aagaagccgg ccttttgggt tggtgacggt    420
   acctgcagaa gaagcgccgg ctaactacgt gccagcagcc gcggtaatac gtagggcgca    480
   agcgttatcc ggaattattg ggcgtaaaga gctcgtaggc ggtttgtcgc gtctgccgtg    540
   aaagtccggg gcttaactcc ggatctgcgg ngggtacggg cagactagag tgcagtaggg    600
   gagactggaa ttcctggtgt agcggtgaaa tgcgcagata tcaggaggaa caccgatggc    660
   gaaggcaggt ntctgggctg taactgacgc tgaggagcga aagcatgggg agcgaacagg    720
   attagatacc ctggtagtcc atgccgtaaa cgttgggcac taggtgtggg ggacattcca    780
   cgttttccgc gccgtagcta acgcattaag tgccccgcct ggggagtacg gccgcaaggc    840
   taaaactcaa aggaattgac gggggcccgc acaagcggcg gagcatgcgg attaattcga    900
   tgcaacgcga agaaccttac caaggcttga catgaaccgg aatgatgcag agatgtgtca    960
   gccacttgtg gccggtttac aggtggtgca tggttgtcgt cagctcgtgt cgtgagatgt   1020
   tgggttaagt cccgcaacga gcgcaaccct cgttccatgt tgccagcggg ttatgccggg   1080
   gactcatggg agactgccgg ggtcaactcg gaggaaggtg gggacgacgt caaatcatca   1140
   tgccccttat gtcttgggct tcacgcatgc tacaatggcc ggtacaaagg gttgcgatac   1200
   tgtgaggtgg agctaatccc aaaaagccgg tctcagttcg gattgaggtc tgcaactcga   1260
   cctcatgaag ttggagtcgc tagtaatcgc agatcagcaa cgctgcggtg aatacgttcc   1320
   cgggccttgt acacaccgcc cgtcaagtca cgaaagtngt aacacccgaa gccggngcct    1380
   aaccccttgn ggagggagcc                                                1400